# EUROPEAN PATENT SPECIFICATION

(11) **EP 0 750 000 B1**
(45) Date of publication and mention of the grant of the patent: **03.05.2000**
(21) Application number: 96202116.8
(22) Date of filing: 14.07.1994
(51) Int. Cl.: C08G 65/30, C08G 65/32, C08G 65/20, C08G 65/10

(54) **Depolymerisation of polyethers using heterogeneous catalysts**
Depolymerisierung von Polyethern in Gegenwart von heterogenen Katalysatoren
Dépolymérisation de polyéthers utilisant des catalyseurs hétérogènes

(30) Priority: 16.07.1993 US 93119
(43) Date of publication of application: 27.12.1996
(62) Divisional of application: 94923920.6
(73) Proprietor: E.I. DU PONT DE NEMOURS AND COMPANY, Wilmington Delaware 19898 (US)
(72) Inventor: Drysdale, Neville Everton, Newark, Delaware 19702-1026 (US); Herron, Norman, Newark, Delaware 19711 (US)
(74) Representative: Jones, Alan John

(56) References cited:
- EP-A- 0 004 536
- EP-A- 0 665 859
- WO-A-94/09055
- DATABASE WPI Derwent Publications Ltd., London, GB; AN 85-181197(30) XP002018925 & JP-A-60 109 584 (ASAHI CHEM IND KK)
- DATABASE WPI Derwent Publications Ltd., London, GB; AN 87-353218(50) XP002018926 & JP-A-62 257 931 (ASAHI CHEMICAL IND KK)

## Description

Polymerisation of cyclic ethers to polyethers is catalysed by heterogenous catalysts which contain selected metal cations. Depolymerisation of polytetrahydrofurans to tetrahydrofurans is catalysed by a heterogenous catalyst containing a metal perfluoroalkylsulfonate, preferably a triflate, attached to the catalyst.

This invention concerns a process for the depolymerization of a polyether to a tetrahydrofuran, comprising contacting at a temperature of about 100°C to about 250°C, a polymer consisting essentially of one or more repeat units of the formula

-[CHR¹CR²R³CR²R³CHR⁴O]-

wherein each R¹, R², R³ and R⁴ is independently hydrogen or hydrocarbyl containing 1 to 20 carbon atoms, with a heterogeneous catalyst containing a metal perfluoroalkylsulfonate attached to the surface of said catalyst, said metal selected from strontium, barium, scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, platinum, copper, silver, gold, zinc, cadmium, mercury, aluminum, gallium, indium, thulium, germanium, tin, lead, arsenic, antimony and bismuth.

The catalyst used herein contains selected metal cations. Preferred metal cations are those of strontium, scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin and bismuth. More preferred metals are yttrium, the rare earth metals, scandium and zirconium. Especially preferred metals are yttrium, ytterbium, dysprosium, erbium, neodymium, lanthanum, scandium and zirconium. Another preferred metal is "mischmetall" (sometimes also called "didymium"), which is a mixture of rare earth metals as obtained from the ore. By rare earths herein is meant lanthanum, cerium, praeseodymium, neodymium, promethium, samarium, europium, gadolinium, terbium, dysprosium, holmium, erbium, thulium, ytterbium, and lutetium. By a perfluoroalkylsulfonate herein is meant a metal salt of a perfluoroalkylsulfonate in which the metal is bonded to one or more perfluoroalkylsulfonate groups.

In the heterogeneous catalyst, a "catalytic" metal perfluoroalkylsulfonate is attached to the surface of a material that in effect acts as a heterogeneous support for the metal perfluoroalkylsulfonate. The metal, in certain embodiments, is not attached to the surface through the perfluoroalkylsulfonate, but through another bond or ligand. The catalytic metal should have at least one perfluoroalkylsulfonate anion attached to it, and preferably, except for the group attaching the metal to the support surface, all of the groups should be perfluoroalkylsulfonate.

The metal may be attached to the surface by a covalent bond, or coordination, or any other method. It is preferred if significant amounts (>25%, preferably <10%) of the catalytic metal cannot be leached from the heterogeneous catalyst by the polymerization process liquids. In one method of attachment, a ligand which can coordinate with the metal cation is attached via one or more covalent bonds to the surface of the support, and then the metal cation is coordinated to the ligand, thereby fixing the metal cation on the support surface. Particularly useful for such are silicon compounds to which the ligand is attached by a stable (to hydrolysis and the polymerization process conditions) bond, and in which the silicon is directly attached to groups which are readily hydrolyzed. When these hydrolytically unstable groups are hydrolyzed from the silicon atom, the resulting "compound" can readily bond to surfaces which have hydroxyl groups present. Many common supports, such as alumina, silica (gel), many metal oxides, and others have such surfaces. After the ligand is attached to the surface, an appropriate metal compound is contacted with the surface containing the ligands, and the metal cation becomes fixed to the support surface. See Examples 1 to 7 for such processes.

The heterogeneous supports for such catalysts can be those which are commonly used for supported catalysts. It is preferred if they have a relatively large surface area, at least 25 m²/g, and it is also preferred if the support is inorganic (inorganic includes various forms of carbon, such as activated carbon, graphite, etc.). Useful supports include alumina, silica, silica-aluminates, carbon, zirconia, yttria, magnesia, ceria, aluminum fluoride and barium sulfate. Preferred supports are alumina, silica, silica-aluminates, carbon and zirconia. It is preferred if the supports themselves are acidic. Although not critical, a convenient amount of the catalytic metal on the catalyst is about 0.1 to about 20 weight percent, measured as catalytic metal.

The depolymerization process is carried out at about 100°C to about 250°C, preferably about 130°C to about 200°C. Although air can be used to blanket the process, it is preferred to use an inert atmosphere such as nitrogen to avoid possible side reactions. A solvent may be used, but it is preferred to carry out the process without solvent.

The amount of catalyst compared to polyether present is not critical, 0.1-15% by weight (percent of the catalyst to polyether) being useful, preferably about 1 to 3% by weight of catalyst.

The depolymerization process may be carried out by just heating the polyether in the presence of the heterogeneous catalyst. In order to avoid boiling off the often volatile tetrahydrofurans, a pressure vessel may be needed. However, it is preferred to carry out the depolymerization while constantly distilling off the (substituted) tetrahydrofuran as it forms. It is believed that this ensures driving this process to produce the monomeric tetrahydrofuran.

In the depolymerisation process the heterogeneous catalyst may be recovered and reused by filtration, and if desired, drying. The recovered catalyst may be used again in the depolymerisation process.

The above process may be carried out as batch, semibatch or continuous processes. Continuous type processes are preferred.

In the Examples, the following abbreviations are used:
- DETM-: diethyl 2-[3-(triethoxysilyl)propyl)]malonate
- GPC -: gel permeation chromatography
- THF -: tetrahydrofuran

### EXAMPLE 1

### Heterogeneous 10 wt% Yttrium triflate on alumina catalyst preparation

25 g commercial alumina pellets AL-3945 (3.2 mm dia x 3.2 mm)was placed in 250 mL water and the pH adjusted to 3 with acetic acid. After stirring 15 mins the pellets were collected by filtration and suction dried. A solution of 190 mL ethanol and 10 mL water had its apparent pH adjusted to 5 with acetic acid and 5 g of DETM was added. After stirring for 5 minutes the alumina pellets were added and agitated for 30 minutes. The supernatant liquid was then decanted and the pellets washed with 2 x 25 mL ethanol and suction dried. The solid was dried in flowing nitrogen by heating to 110°C for 1 hour and then sealed and taken into a nitrogen glove box. 5 g yttrium triflate was dissolved in 50 mL acetonitrile and added to the dry alumina pellets. This slurry was allowed to sit undisturbed overnight in the glove box and then evaporated to dryness in vacuo. The solid was then extracted with 2 x 25 mL acetonitrile, filtered and washed with acetonitrile. Evaporation of all washings and extracts indicated that ∼50% of the original yttrium triflate had been retained on the pellets. The pellets were suction dried and then dried in flowing nitrogen at 110°C for 1 hour before returning to the glove box for collection and storage prior to testing.

### EXAMPLE 2

### Heterogeneous 10 wt% Yttrium triflate on silica-alumina catalyst preparation

25 g commercial silica-alumina pellets (Alfa cat # 31269; 91% Al₂O₃, 6% SiO₂) was placed in 250 mL water and the pH adjusted to 3 with acetic acid. The pellets were then treated in a manner identical to that described in Example 1 above.

### EXAMPLE 3

### Heterogeneous 10 wt% Zirconium triflate on alumina catalyst preparation.

25 g commercial alumina pellets (Al-3945, 3.2 mm dia. x 3.2 mm)) was placed in 250 mL water and the pH adjusted to 3 with acetic acid. The pellets were then treated in a manner identical to that described in Example 1 above except substituting zirconium triflate for yttrium triflate.

### EXAMPLE 4

### Preparation of Yttrium triflate supported on alumina derivatized with diethylmalonate

95 mL ethanol and 5 mL water were mixed and the pH adjusted to 5 with acetic acid. 2 g DETM was added and the mix stirred for 5 minutes. 10 g γ-alumina was added and the slurry stirred a further 3 min. The solid was allowed to settle and the supernatant liquid decanted. The recovered solid was then washed with two portions of 25 mL ethanol and suction dried. The solid was finally dried in flowing nitrogen (200 mL/min) by heating to 110°C and holding at that temperature for 1 hour. The cooled powder was immediately transferred to a nitrogen glove box.

Under a dry nitrogen atmosphere inside a glove box, 0.5 g yttrium triflate was dissolved in 25 mL acetonitrile and 5 g of the alumina powder prepared above was added. The slurry was stirred for 1 hour and then filtered, rinsed with 25 mL acetonitrile and suction dried. The powder was then pumped to dryness in vacuum and stored under nitrogen.

### EXAMPLE 5

### Preparation of bis(n-cyclopentadienyl)tetra-hydrofuran-bis(trifluoromethanesulfonato)-zirconium supported on silica derivatized with DETM

95 mL ethanol and 5 mL water were mixed and the pH adjusted to 5 with acetic acid. 2 g DETM was added and the mix stirred for 5 minutes. 10 g silica was added and the slurry stirred a further 3 mins. The solid was allowed to settle and the supernatant liquid decanted. The recovered solid was then washed with two portions of 25 mL ethanol and suction dried. The solid was finally dried in flowing nitrogen (200 mL/min) by heating to 110°C and holding at that temperature for 1 hour. The cooled powder was immediately transferred to a nitrogen glove box.

Under a dry nitrogen atmosphere inside a glove box, 0.5 g bis(n-cyclopentadienyl)tetrahydrofuran-bis(trifluoromethanesulfonato)zirconium was dissolved in 25 mL acetonitrile and 5 g of the silica powder was added. The slurry was stirred for 1 hour and then filtered, rinsed with 25 mL acetonitrile and suction dried. The powder was then pumped to dryness in vacuum and stored under nitrogen.

### EXAMPLE 6

### Preparation of bis(n-cyclopentadienyl)tetrahydrofuran-bis(trifluoromethanesulfonato)zirconium supported on alumina derivatized with DETM

95mL ethanol and 5 mL water were mixed and the pH adjusted to 5 with acetic acid. 2 g DETM was added and the mix stirred for 5 minutes. 10 g λ-alumina was added and the slurry stirred a further 3 mins. The solid was allowed to settle and the supernatant liquid decanted. The recovered solid was then washed with two portions of 25 mL ethanol and suction dried. The solid was finally dried in flowing nitrogen (200 mL/min) by heating to 110°C and holding at that temperature for 1 hour. The cooled powder was immediately transferred to a nitrogen glove box.

Under a dry nitrogen atmosphere inside a glove box, 0.5 g bis (n-cyclopentadienyl)tetrahydrofuran-bis (trifluoromethanesulfonato)zirconium was dissolved in 25 mL acetonitrile and 5 g of the alumina powder prepared above was added. The slurry was stirred for 1 hour and then filtered, rinsed with 25 mL acetonitrile and suction dried. The powder was then pumped to dryness in vacuum and stored under nitrogen.

### EXAMPLE 7

### Preparation of ∼10 wt% Ytterbium triflate supported on silica-alumina

50 g silica-alumina pellets (3.2 mm dia. x 3.2 mm) were placed in 250 mL distilled water. The pH was adjusted to 3 with acetic acid and the slurry was stirred for 15 mins. The pellets were collected by filtration and then added to a solution of 190 mL ethanol, 10 mL water in which the pH was adjusted to 5 with acetic acid and then 5 g DETM was added. Stirred for 30 min and then the supernatant liquid was decanted. The pellets were washed with two portions of 25 mL ethanol and then suction dried prior to drying in flowing nitrogen (200 mL/min) at 110°C for 1 hour. The pellets were then transferred to a nitrogen glove box. Inside the glove box 10 g ytterbium triflate was dissolved in 100 mL dry acetonitrile and this solution was added to the dry pellets. The slurry then sat overnight under nitrogen before being evaporated to dryness. The recovered solid was washed with three 25 mL portions of dry acetonitrile, suction dried and then dried in flowing nitrogen to 110°C for 1 hour. The dry pellets were stored under nitrogen in a glove box.

### EXAMPLE 8

### Depolymerization of Polytetrahydrofuran (Terathane® 1000) with 10% Ytterbium Triflate Supported on Silica-Alumina

Polytetrahydrofuran with hydroxyl ends (Terathane® 1000, 300 g, Aldrich) and the 10% ytterbium triflate supported on silica-alumina of Example 7 (10 g) were placed in a 500 mL three neck flask equipped with a stirring bar, Vigreaux column (30.5 cm(12")) and a fractional distillation head. A nitrogen purge was attached and all other openings were glass stoppered. The resulting mixture was heated with an oil bath and the resulting water clear distillate fractions collected as follows:

| Fraction | Oil Bath Temp (°C) | Rxn Temp (°C) | Head Temp (°C) | Weight (g) |
|---|---|---|---|---|
| 1 | 180 | 152 | 64 | 70.72 |
| 2 | 198 | 163 | 66 | 112.0 |
| 3 | 196 | 124 | 66 | 59.00 |
| 4 | 202 | 149 | 66 | 55.00 |

Total weight of distillate collected: 296.72 g
% Yield (Recovery): 98.9%
GC analyses of the various fractions confirm the product to be THF

### COMPARATIVE EXAMPLE 1

In a dry box, zeolite HY (1.00 g) was added to an oven dry 100 mL RB flask equipped with a stirring bar. The flask was sealed with a rubber septum and removed from the dry box. After the attachment of a nitrogen bleed THF (20.00 mL) was added by syringe. After 45 minutes acetic anhydride (1.00 mL) was added. After filtering off the zeolite catalyst and concentration of the filtrate, no polymer was obtained.

## Claims

1. A process for the depolymerisation of a polyether to a tetrahydrofuran, comprising contacting at a temperature of 100°C to 250°C, a polymer consisting essentially of one or more repeat units of the formula
-[CHR¹CR²R³CR²R³CHR⁴O]-
wherein each R¹, R², R³ and R⁴ is independently hydrogen or hydrocarbyl containing 1 to 20 carbon atoms, with a heterogeneous catalyst containing a metal perfluoroalkylsulfonate attached to the surface of said catalyst, said metal selected from strontium, barium, scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, platinum, copper, silver, gold, zinc, cadmium, mercury, aluminium, gallium, indium, thulium, germanium, tin, lead, arsenic, antimony and bismuth.

2. The process as recited in claim 1 wherein R¹, one of R², both of R³ and R⁴ are hydrogen, and the remaining R² is alkyl containing 1-4 carbon atoms.

3. The process as recited in claim 1 wherein all of R¹, R², R³ and R⁴ are hydrogen.

4. The process as recited in any one of the preceding claims wherein said metal cation is strontium, scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin or bismuth.

5. The process as recited in any one of the preceding claims wherein said heterogeneous catalyst support is alumina, silica, silica-aluminate, carbon or zirconia.

6. The process as recited in any one of the preceding claims wherein said heterogeneous catalyst is such that the metal is attached to the surface not through the perfluoroalkysulfonate but through another bond or ligand.

## Patentansprüche

1. Verfahren zur Depolymerisierung eines Polyethers zu einem Tetrahydrofuran, umfassend die Schritte, daß bei einer Temperatur von 100°C bis 250°C ein Polymer, das im wesentlichen aus einer oder mehreren sich wiederholenden Einheiten der Formel
-[CHR¹CR²R³CR²R³CHR⁴O]-
besteht, wobei jedes R¹, R², R³ und R⁴ unabhängig voneinander Wasserstoff oder ein Kohlenwasserstoffrest ist, der 1 bis 20 Kohlenstoffatome enthält,
mit einem heterogenen Katalysator in Kontakt gebracht wird, der ein Metall-Perfluoralkylsufonat enthält, das an die Oberfläche des Katalysators gebunden ist, wobei das Metall ausgewählt ist aus Strontium, Barium, Scandium, Yttrium, den Seltenerdmetallen, Titan, Zirkonium, Hafnium, Vanadium, Niob, Chrom, Molybdän, Tantal, Wolfram, Rhenium, Eisen, Ruthenium, Osmium, Rhodium, Iridium, Palladium, Platin, Kupfer, Silber, Gold, Zink, Cadmium, Quecksilber, Aluminium, Gallium, Indium, Thulium, Germanium, Zinn, Blei, Arsen, Antimon und Wismut.

2. Verfahren nach Anspruch 1, wobei R¹, eines von R², beide von R³ sowie R⁴ Wasserstoff sind und das verbleibende R² ein Alkylrest ist, der 1 - 4 Kohlenstoffatome enthält.

3. Verfahren nach Anspruch 1, wobei alle R¹, R², R³ und R⁴ Wasserstoff sind.

4. Verfahren nach einem der vorhergehenden Ansprüche, wobei das Metallkation Strontium, Scandium, Yttrium, ein Seltenerdmetall, Titan, Zirkonium, Hafnium, Vandium, Niob, Tantal, Chrom, Molybdän, Wolfram, Rhenium, Eisen, Ruthenium, Palladium, Kupfer, Gold, Zink, Zinn oder Wismut ist.

5. Verfahren nach einem der vorhergehenden Ansprüche, wobei der heterogene Katalysatorträger Aluminiumoxid, Siliciumdioxid, Siliciumdioxid-Aluminat, Kohlenstoff oder Zirkoniumdioxid ist.

6. Verfahren nach einem der vorhergehenden Ansprüche, wobei der heterogene Katalysator so ist, daß das Metall nicht durch das Perfluoralkylsulfonat an die Oberfläche gebunden ist, sondern durch eine andere Bindung oder einen anderen Liganden.

## Revendications

1. Procédé pour la dépolymérisation d'un polyéther en un tétrahydrofuranne, comprenant la mise en contact à une température de 100°C à 250°C, d'un polymère constitué essentiellement d'une ou de plusieurs unités répétitives de la formule
-[CHR¹CR²R³CR²R³CHR⁴O]-
dans laquelle chaque R¹, R², R³ et R⁴ est indépendamment un hydrogène ou un hydrocarbure contenant de 1 à 20 atomes de carbone, avec un catalyseur hétérogène contenant un perfluoroalkylsulfonate métallique attaché à la surface dudit catalyseur, ledit métal sélectionné parmi du strontium, du baryum, du scandium, de l'yttrium, les métaux terres rares, du titane, du zirconium, de l'hafnium, du vanadium, du niobium, du chrome, du molybdène, du tantale, du tungstène, du rhénium, du fer, du ruthénium, de l'osmium, du rhodium, de l'iridium, du palladium, du platine, du cuivre, de l'argent, de l'or, du zinc, du cadmium, du mercure, de l'aluminium, du gallium, de l'indium, du thulium, du germanium, de l'étain, du plomb, de l'arsenic, de l'antimoine et du bismuth.

2. Procédé comme décrit à la revendication 1, dans lequel R¹, un des R², à la fois R³ et R⁴ sont un hydrogène, et le R² restant est un alkyle contenant 1-4 atomes de carbone.

3. Procédé comme décrit à la revendication 1, dans lequel tous les R¹, R², R³ et R⁴ sont un hydrogène.

4. Procédé comme décrit à l'une quelconque des revendications précédentes, dans lequel ledit cation métallique est du strontium, du scandium, de l'yttrium, les métaux terres rares, du titane, du zirconium, de l'hafnium, du vanadium, du niobium, du tantale, du chrome, du molybdène, du tungstène, du rhénium, du fer, du ruthénium, du palladium, du cuivre, de l'or, du zinc, de l'étain ou du bismuth.

5. Procédé comme décrit à l'une quelconque des revendications précédentes, dans lequel ledit support de catalyseur hétérogène est de l'alumine, de la silice, de la silice-alumine, du carbone ou de la zircone.

6. Procédé comme décrit à l'une quelconque des revendications précédentes, dans lequel ledit catalyseur hétérogène est tel que le métal est attaché à la surface non pas par le perfluoroalkylsulfonate mais par une autre liaison ou un autre ligand.
